# EUROPEAN PATENT SPECIFICATION

(11) **EP 4 082 491 B1**
(45) Date of publication and mention of the grant of the patent: **02.07.2025**
(21) Application number: 20905091.3
(22) Date of filing: 04.09.2020
(51) Int. Cl.: A61F 2/966, A61F 2/95

(54) **IMPLANT DELIVERY SYSTEM**
IMPLANTATEINFÜHRUNGSSYSTEM
SYSTÈME DE POSE D'IMPLANT

(30) Priority: 24.12.2019 CN 201911349231
(43) Date of publication of application: 02.11.2022
(73) Proprietor: Shanghai Bluevascular Medtech Co., Ltd., Shanghai 201321 (CN)
(72) Inventor: FAN, Yaming, Shanghai 201321 (CN); ZHANG, Zhaoduo, Shanghai 201321 (CN); ZHANG, Junli, Shanghai 201321 (CN); WANG, Liwen, Shanghai 201321 (CN); ZHANG, Linlin, Shanghai 201321 (CN); ZHU, Qing, Shanghai 201321 (CN)
(74) Representative: Lavoix
(86) International application number: PCT/CN2020/113615
(87) International publication number: WO 2021/128940

(56) References cited:
- CN-A- 106 691 648
- CN-A- 108 403 269
- CN-A- 109 248 012
- CN-A- 110 882 096
- CN-U- 209 075 051
- CN-U- 209 075 052
- US-A1- 2005 240 210
- US-A1- 2006 142 694
- US-A1- 2011 270 372
- US-A1- 2014 046 428
- US-A1- 2019 307 588

## Description

### TECHNICAL FIELD

The present invention relates to the technical field of medical devices and, more specifically, to an implant delivery system.

### BACKGROUND

In the field of interventional medicine, the technique using implants to treat or relieve stenosis and occlusive lesions in the body's lumen is more and more widely used in clinical practice, such as the treatment of iliac vein compression syndrome. Intraluminal implants are generally compressible implants such as self-expanding implants. The implants are configured to be compressed into a hollow catheter. Under the supervision using digital imaging (such as DSA, CT, ultrasound, endoscopy, etc.), the implant compressed in the catheter is delivered to the diseased area, and then the implant is released and expanded in a certain way. Relying on the radial support force of the implant itself and the contraction force of the body lumen, the implant is fixed in a specific position to achieve the purpose of treating or alleviating the disease.

Self-expanding implants may fall into two categories, one will be shortened in length when expanding (e.g., woven implants), and the other does not change in length when expanding (e.g., laser engraved implants). The current delivery system of this type of implant usually includes: at least one outer tube configured for compressing the implant in it; a jacking tube pre-installed in the outer tube with its position fixed, and the jacking tube has a distal end configured to "hold" the implant in order to prevent the implant from moving proximally with the outer tube when the outer tube is withdrawn; and an inner tube is configured for connecting the distal guide member (e.g., a front end component with a taper) and for receiving the guide wire. The method of delivering and placing a self-expanding implant generally includes the following steps: the delivery system pre-installed with a implant is pushed to the lesion site under the guidance of the guide wire; the delivery system is then fine-tuned according to the visualization marking points on the distal end of the implant, so as to determine the position of the implant to be released; then the jacking tube is fixed, and the outer tube is withdrawn to release the implant from the outer tube; and finally the implant is expanded by its own expansion force.

During the release process of the above-mentioned implant, the length of the implant will be shortened as the implant expands, resulting in a displacement which causes the implant to not accurately reach the predetermined position. As a result, the diseased blood vessel cannot be fully supported by the implant. There is still a stenosis or obstruction remaining. The expected therapeutic purpose can not be achieved.

US 2014/046428 A1 discloses handle assemblies for stent graft delivery systems.

### SUMMARY OF THE INVENTION

An objective of the present invention is to provide an implant delivery system, which is able to compensate for the shortening and displacement of the implant caused by expansion during the process of releasing the implant, so that the implant can reach a predetermined position to ensure the treatment effect.

In order to achieve the above objective, the present invention provides an implant delivery system according to claim 1, comprising: a handle, a first inner sliding member, a second inner sliding member, an inner tube, an outer tube, and a jacking tube, wherein the handle has a first cavity in which the first inner sliding member and the second inner sliding member are located;
the outer tube has a first proximal end and a first distal end opposite to each other, and the first proximal end is connected to the first inner sliding member; the jacking tube has a second proximal end and a second distal end opposite to each other, the second distal end is arranged in the outer tube, and the second proximal end is connected to the second inner sliding member; the inner tube extends through the outer tube and the jacking tube, and a cavity configured to receive a implant is formed between the inner tube and the outer tube; and the second distal end is configured for connecting with a proximal part of the implant;
the handle is threadedly matched with the first inner sliding member and the second inner sliding member for screw transmission, and configured to drive the first inner sliding member and the second inner sliding member to move linearly along an axis of the handle in opposite directions so as to drive the outer tube and the jacking tube to move in the opposite directions to release the implant from a distal end to a proximal end thereof.

Optionally, an outer surface of the first inner sliding member is provided with a first external thread, and an outer surface of the second inner sliding member is provided with a second external thread, thread directions of the first external thread and the second external thread are opposite.

Optionally, an inner surface of the handle is provided with a first internal thread and a second internal thread, and the first internal thread of the handle is threadedly matched with the first external thread of the first inner sliding member for screw transmission, and the second internal thread of the handle is threadedly matched with the second external thread of the second inner sliding member for screw transmission.

Optionally, the handle comprises two sub-handles having inner cavities, and each of the inner cavities has a semicircular arc shape on a cross section perpendicular to an axial direction of the handle.

Optionally, each of the first inner sliding member and the second inner sliding member has an arc shape on a cross section perpendicular to the axis of the handle, and a concave surface of the first inner sliding member is arranged opposite to a concave surface of the second inner sliding member.

Optionally, each of the first inner sliding member and the second inner sliding member has a semicircular arc shape on a cross section perpendicular to the axis of the handle.

A positioning groove extending along an axial direction of the handle is defined in each of the first inner sliding member and the second inner sliding member, and the first inner sliding member and the second inner sliding member are slidably connected to an arc-shaped plate through the positioning groove.

Optionally, the first inner sliding member has two first end surfaces extending along the axial direction of the handle, the positioning groove is defined in each of the two first end surfaces, and the first inner sliding member is slidably connected to the two arc-shaped plates through the two positioning grooves; the second inner sliding member has two second end surfaces extending along the axial direction of the handle, the positioning groove is further defined in each of the two second end surfaces, and the second inner sliding member is slidably connected to the two arc-shaped plates through the two positioning grooves.

Optionally, the first end surface is provided with a raised limiting member, the second end surface is defined with a sliding groove extending along the axial direction of the handle, and the limiting member is installed in the sliding groove; or, the first end surface is defined with a sliding groove, the second end surface is provided with a raised limiting member extending along the axial direction of the handle, and the limiting member is installed in the sliding groove.

The implant delivery system further comprises a proximal connector and a distal connector provided at respective ends of the handle, the proximal connector and the distal connector are connected by two arc-shaped plates having concave surfaces which are arranged opposite to each other and define a second cavity; the distal connector is defined with a first through hole penetrating in an axial direction; the proximal connector is defined with a second through hole penetrating in the axial direction, and the inner tube extends through the first through hole, the second cavity and the second through hole.

Optionally, the arc-shaped plate has a third proximal end and a third distal end opposite to each other; a first blind hole is defined in the proximal connector, and the third proximal end of the arc-shaped plate is installed in the first blind hole; a second blind hole is defined in the distal connector, and the third distal end of the arc-shaped plate is installed in the second blind hole.

Optionally, the implant delivery system further comprises a head of the jacking tube arranged at the second distal end of the jacking tube, and the head is provided with a holding structure for holding and releasing the implant.

Optionally, the holding structure comprises a plurality of hooks spaced apart along a circumferential direction of the head of the jacking tube.

Optionally, the implant delivery system further comprises a guide member, and the inner tube has a fourth proximal end and a fourth distal end opposite to each other, the fourth distal end of the inner tube protrudes from the first distal end of the outer tube and is connected to the guide member.

Optionally, the implant delivery system further comprises at least a developing ring arranged at the fourth distal end of the inner tube.

Optionally, the first proximal end of the outer tube is connected to an outer wall of the jacking tube through an outer tube connection assembly, and the outer tube is slidable along the outer wall of the jacking tube.

Optionally, the outer tube connection assembly comprises an outer tube connector, a first sealing ring and a first sealing cap; the outer tube connector has a first connection hole whose one end is fixedly connected to the outer tube, a further end is sleeved over the jacking tube; and the first sealing ring is sleeved over the jacking tube and is in sealing connection with the outer tube when the first sealing cap is tightened.

Optionally, the outer tube connector is further defined with a first snap groove, the first inner sliding member is provided with a first snap ring, and the first snap groove is detachably connected to the first snap ring.

Optionally, the second proximal end of the jacking tube is provided with a jacking tube connection assembly comprising a jacking tube connector, wherein the jacking tube connector is arranged at the second proximal end of the jacking tube, and the jacking tube connector is defined with a second connecting hole through which the jacking tube connector is sleeved over the inner tube and is slidable along the inner tube.

Optionally, the jacking tube connection assembly further comprises a second sealing ring and a second sealing cap, the second sealing ring is sleeved over the inner tube, the second sealing cap is used to fasten the second sealing ring so that the jacking tube connector is in sealing connection with the inner tube.

Optionally, the jacking tube connector is defined with a second snap groove, the second inner sliding member is provided with a second snap ring, and the second snap groove is detachably connected to the second snap ring.

Optionally, the implant delivery system further comprises a first infusion tube and a second infusion tube; the fourth proximal end of the inner tube protrudes from the second proximal end of the jacking tube and is in communication with the first infusion tube; the outer tube connector is provided with a first branch connector, and a third connecting hole is defined in the first branch connector and is in communication with the second infusion tube; or,
the tube jacking tube connector is provided with a second branch connector, and a fourth connecting hole is defined in the second branch connector and is in communication with the second infusion tube.

Optionally, the fourth proximal end of the inner tube is provided with an inner tube connection assembly comprising a first connecting sleeve, a second connecting sleeve and a locking cap; the first connecting sleeve is used to connect the inner tube and the first infusion tube, the second connecting sleeve is sleeved over the first connecting sleeve and is used to fix the fourth proximal end of the inner tube, and the locking cap is used to lock the second connecting sleeve, the first connecting sleeve and the first infusion tube in position.

Optionally, the first inner sliding member and the second inner sliding member are shaped as two hollow tube-shaped structures arranged coaxially with each other.

Compared with the prior art, the present invention offers the following advantages.
1. The implant delivery system of the present invention includes a handle, a first inner sliding member, a second inner sliding member, an inner tube, an outer tube, and a jacking tube. Both the first inner sliding member and the second inner sliding member are threadedly matched with the handle for screw transmission. When the handle is rotated relative to the first inner sliding member and the second inner sliding member, the first inner sliding member and the second inner sliding member are simultaneously driven to move linearly along the axis of the handle in opposite directions, thereby driving the outer tube and the jacking tube to move in the opposite directions. As a result, during the release of the implant, the first inner sliding member drives the outer tube to withdraw so that the implant is released out of the outer tube from its distal end to its proximal end, at the same time, the second inner sliding member drives the jacking tube to move to a predetermined position, thereby pushing the implant to move to a predetermined position to compensate for the displacement casued by the shortening of the implant due to expansion; the outer tube and the jacking tube are driven to move by means of screw feeding, which can accurately control the moving distances of the outer tube and the jacking tube, and then accurately deliver the implant to a predetermined position to ensure treatment effect.
2. The first inner sliding member and the second inner sliding member are provided with external threads having opposite thread directions, and both the first inner sliding member and the second inner sliding member are structured to have a semicircular arc shape, and they at least partially overlap in the axial direction, which can shorten the length of the handle, facilitate operation, and reduce the size of the implant delivery system.

### BRIEF DESCRIPTION OF THE DRAWINGS

Fig. 1 is a structural schematic of an implant delivery system in accordance with an embodiment of the present invention;
Fig. 2 is a structural schematic of a first inner slidable member in the implant delivery system in accordance with an embodiment of the present invention;
Fig. 3 is a structural schematic of a second inner sliding member in the implant delivery system in accordance with an embodiment of the present invention;
Fig. 4 is a schematic diagram showing the connection between the first inner sliding member, the second inner sliding member, and the circumferential positioning member in accordance with an embodiment of the present invention;
Figs. 5a and 5b are schematic diagrams of the disassembly and assembly of the handle with the first inner sliding member and the second inner sliding member in accordance with an embodiment of the present invention, wherein one sub-handle is assembled in Fig. 5a, and both of the two sub-handles of the handle are assembled in Fig. 5b;
Fig. 6 is a schematic diagram showing the connection between the first distal end, the second distal end, the fourth distal end, the guide member, and the implant in the implant delivery system in accordance with an embodiment of the present invention;
Fig. 7 is a schematic diagram showing the connection of the inner tube with the guide member and the first infusion tube in the implant delivery system in accordance with an embodiment of the present invention;
Fig. 8 is a structural schematic of an end portion of the jacking tube in the implant delivery system in accordance with an embodiment of the present invention;
Fig. 9 is a schematic diagram showing the connection of the second proximal end of the jacking tube with the inner tube and the second infusion tube in the implant delivery system in accordance with an embodiment of the present invention;
Fig. 10 is a schematic diagram showing showing the connection between the first proximal end of the outer tube and the jacking tube in the implant delivery system in accordance with an embodiment of the present invention;
Fig. 11 is a schematic diagram showing the connection of the handle with the first inner sliding member and the second inner sliding member in accordance with another embodiment of the present invention.

In the drawings:
10 stent
110 handle
111 sub-handle
120 first inner sliding member;
121 first external thread, 122 raised limiting member, 123 first snap ring;
130 second inner sliding member;
131 second external thread, 132 sliding groove, 133 second snap ring;
200 outer tube;
210 outer tube connection assembly;
211 outer tube connector, 212 first sealing ring, 213 first sealing cap;
300 jacking tube;
310 head of the jacking tube;
311 hook;
320 jacking tube connection assembly;
321 jacking tube connector, 322 second sealing ring, 323 second sealing cap, 324 second branch connector;
400 circumferential positioning member;
410 arc-shaped plate, 420 positioning groove, 430 proximal connector, 440 distal connector;
500 inner tube;
510 inner tube connection assembly;
511 first connecting sleeve, 512 second connecting sleeve, 513 locking cap;
520 developing ring;
600 guide member;
710 first infusion tube, 720 Luer taper;
810 second infusion tube, 820 check valve.

### DETAILED DESCRIPTION

The objects, advantages and features of the invention will be more apparent upon reading the following detailed description of the proposed implant delivery system in connection with the accompanying figures. Note that the figures are much simplified and may not be drawn to scale, and their sole purpose is to facilitate easy and clear explanation of some embodiments of the invention.

As used herein and in the appended claims, the singular forms of "a", "an", and "the", include plural references unless the context clearly dictates otherwise. As used in this specification and the appended claims, the term "or" is generally used to include the meaning of "and/or", unless the content clearly indicates otherwise; the terms "installed", "connected", "coupled" should be understood in a broad sense. For example, it can be a fixed connection, a detachable connection, or an integral connection, a mechanical connection, an electrical connection, a direct connection, or an indirect connection through an intermediate medium. It can be a communication between two elements or an interaction relationship between two elements. For those of ordinary skill in the art, the specific meaning of the above-mentioned terms in the present invention can be understood according to specific circumstances. Throughout the figures, same or analogous reference numerals indicate the same or analogous components or elements.

In the present invention, the terms "proximal" and "distal" refer to the relative position, relative orientation, and direction of elements or actions relative to each other from the perspective of the operator using the implant delivery system, although the "distal" and "Proximal" is not restrictive, "proximal end" usually refers to the end of the implant delivery system close to the operator during normal operation, and "distal" usually refers to the end away from the operator, that is, the end that first enters the patient's body.

The implant delivery system provided by the embodiment of the present invention is configured to deliver an implant into a diseased lumen such as a blood vessel. In this embodiment, the stent 10 is used as an implant to be delivered for description, but it should not constitute a limitation to the present invention.

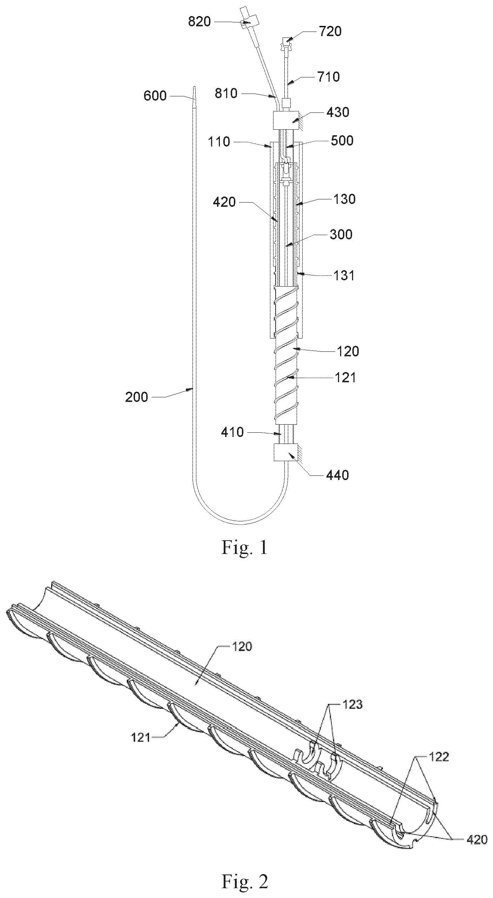

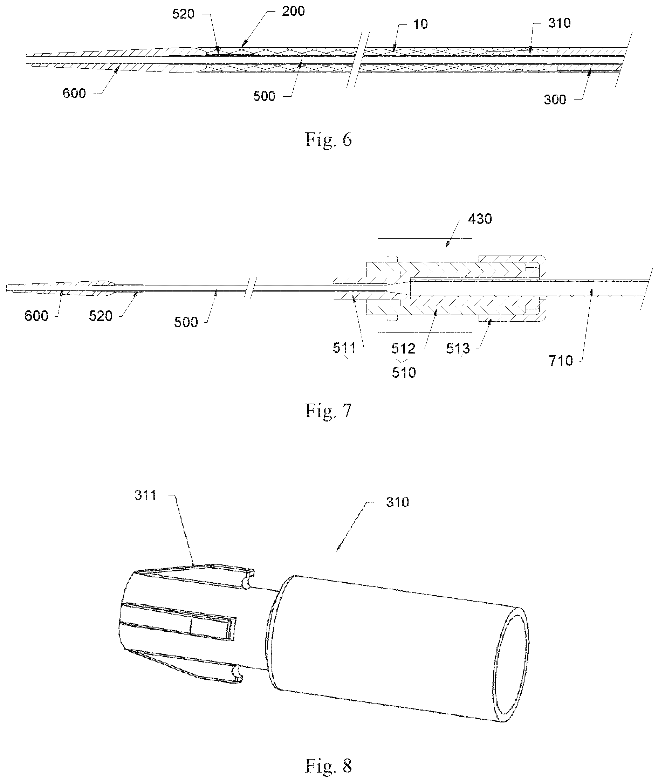

As shown in Figs. 1 and 6, the implant delivery system includes a handle 110, a first inner sliding member 120, a second inner sliding member 130, an outer tube 200, and a jacking tube 300. The handle 110 has a first cavity. The first inner sliding member 120 and the second inner sliding member 130 are located in the first cavity. The outer tube 200 has a first proximal end and a first distal end opposite to each other. The first proximal end is connected to the first inner sliding member 120. The jacking tube has a second proximal end and a second distal end opposite to each other. The second distal end is disposed in the outer tube, and the second proximal end is connected to the second inner sliding member 130.

The handle 110 is threadedly matched with the first inner sliding member 120 and the second inner sliding member 130 for screw transmission, and is configured to drive the first inner sliding member 120 and the second inner sliding member 130 to move linearly along the axis of the handle 110 in two opposite directions, thereby driving the outer tube 200 and the jacking tube 300 to move in the two opposite directions.

The stent 10 is delivered and released by the implant delivery system provided by the present invention. When the handle 110 is rotated, the first inner sliding member 120 can drive the outer tube 200 to withdraw by means of screw transmission, and the stent 10 is released with its volume expanded but length shortened, which results in a deviation from the predetermined position. At the same time, the second inner sliding member 130 drives the jacking tube 300 to move to the predetermined position, thereby pushing the stent 10 to the predetermined position, so that the stent 10 is positioned accurately to ensure a treatment effect.

In the process of releasing the stent 10, in order to ensure that the first inner sliding member 120 and the second inner sliding member 130 move linearly when the handle 110 is lotating, the implant delivery system further includes a circumferential positioning member 400. The circumferential positioning member 400 is used to keep the first inner sliding member 120 and the second inner sliding member 130 fixed in the circumferential direction and moveable in the axial direction.

In addition, when the stent 10 is released, the retracting distance of the outer tube 200 and the pushing distance of the jacking tube 300 need to meet a certain ratio, so that the stent 10 can accurately reach the predetermined position. The retracting distance of the outer tube 200 and the pushing distance of the jacking tube 300 can be calculated through simulation experiments. During operation, the retreating distance of the outer tube 200 is equal to the moving distance of the first inner sliding member 120, and the pushing distance of the jacking tube 300 is equal to the moving distance of the second inner sliding member 130. The moving distances of the first inner sliding member 120 and the second inner sliding member 130 rely on the rotation speed and the pitch. In the present invention, the first inner sliding member 120 and the second inner sliding member 130 are controlled to rotate by the same handle 110, and both have the same rotation speed. The moving distances of the first inner sliding member 120 and the second inner sliding member 130 are respectively determined by the respective pitch. By setting the pitch ratio of the first inner sliding member 120 and the second inner sliding member 130, the first inner sliding member 120 and the second inner sliding member 130 can move according to an expected speed ratio, so that the retreating distance of the outer tube 200 and the pushing distance of the jacking tube 300 meets an expected ratio, so that the stent 10 can accurately reach the predetermined position. For those skilled in the art, it is common knowledge to set the pitch ratio of the two threaded segments according to the ratio of the known moving distances, so it will not be described in detail here.

Next, the first inner sliding member 120, the second inner sliding member 130, and the handle 110 will be further described and illustrated in detail, but not limited to the structure described in the following embodiments.

Reference is made to Figs. 1 to 4, in an embodiment, the outer surface of the first inner sliding member 120 is provided with a first external thread 121, and the outer surface of the second inner sliding member is provided with a second external thread 131. The thread directions of the first external thread 121 and the second external thread 131 are opposite. In a plane perpendicular to the axial direction of the handle 110, the first inner sliding member 120 may have an arc shape, preferably a semicircular arc shape, and the first external thread 121 is provided on the convex surface of the first inner sliding member 120. In a plane perpendicular to the axial direction of the handle 110, the second inner sliding member 130 may have an arc shape, preferably a semicircular arc shape. In this embodiment, the first inner sliding member 120 and the second inner sliding member 130 are both of semicircular arc shapes. The outer diameter of the second inner sliding member 130 is equal in length to the outer diameter of the first inner sliding member 120, and the inner diameters of the second inner sliding member 130 and the first inner sliding member 120 are also equal in length. In other embodiments of the present invention, the first inner sliding member and the second inner sliding member may have other shapes. In other embodiments of the present invention, the outer diameters of the first inner sliding member and the second inner sliding member can be the same or different. In this embodiment, the second external thread 131 is provided on the convex surface of the second inner sliding member 130. The concave surface of the first inner sliding member 120 and the concave surface of the second inner sliding member 130 are arranged opposite to each other (that is, when the first inner sliding member 120 and the second inner sliding member 130 are projected to a plane perpendicular to the axial direction of the handle 110, the projections of the first inner sliding member 120 and the second inner sliding member 130 may form a circle). The first inner sliding member 120 and the second inner sliding member 130 are slidable relatively along the axial direction of the handle 110. During the sliding process, the first inner sliding member 120 and the second inner sliding member 130 may overlap at least partially. This structural design can reduce the axial length of the handle 110, reduce the size of the delivery system, and also make it easier to operate.

Further, the first inner sliding member 120 has two first end surfaces extending along the axial direction of the handle 110, and each of the first end surface may be provided with a raised limiting member 122 extending along the axial direction of the handle 110. The second inner sliding member 130 has two second end surfaces extending along the axial direction of the handle 110, and each of the second end surfaces may be correspondingly provided with a sliding groove 132 extending along the axial direction of the handle 110. The raised limiting member 122 is installed in the sliding groove 132 and is slidable along the sliding groove 132 to realize the circumferential positioning of the first inner sliding member 120 and the second inner sliding member 130. The positions of the raised limiting member 122 and the sliding groove 132 can also be exchanged. That is, the raised limiting member 122 is arranged on the second end surfaces of the second inner sliding member 130, and the sliding groove 132 is arranged on the first end surfaces of the first inner sliding member 120. The raised limiting member 122 can be a continuous member extending along the axial direction of the handle 110, or can be a plurality of members spaced apart along the axial direction of the handle 110. The first inner sliding member 120 can be provided with a raised limiting member on one of the first end surfaces, or can be provided with raised limiting members 122 on both of the first end surfaces; accordingly, the second inner sliding member 130 can be provided with a sliding groove 132 on one of the second end surfaces, or can be provided with sliding grooves on both of the second end surfaces. In some embodiments, the raised limiting member and sliding grooves may not be provided, but the circumferential positioning between the first inner sliding member and the second inner sliding member is realized through specific structures.

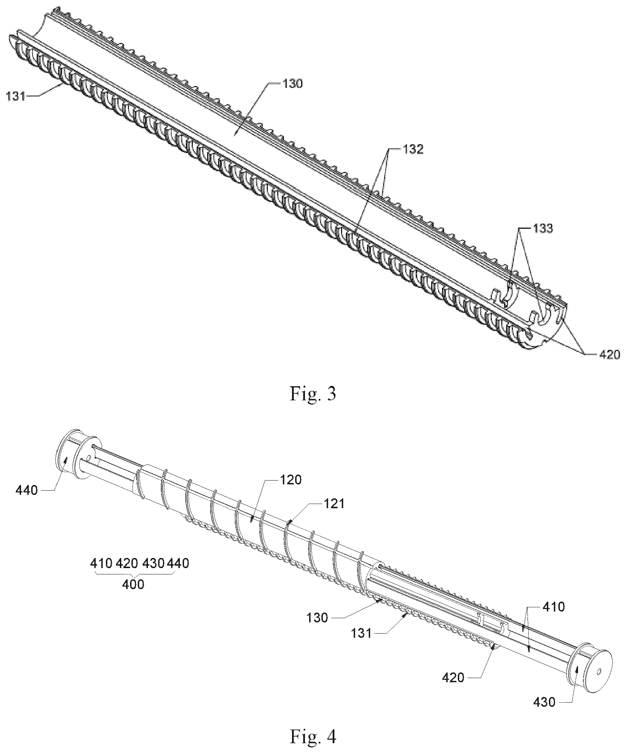

Please continue to refer to Figs. 1 and 4, the circumferential positioning member 400 further includes positioning grooves 420 defined in the first inner sliding member 120 and the second inner sliding member 130, and the positioning grooves 420 extends along the axial direction of the handle 110. The first inner sliding member 120 and the second inner sliding member 130 are mounted to the arc-shaped plate 410 through the positioning grooves 420 and are slidable along the arc-shaped plate 410. As shown in Fig. 4, preferably, two arc-shaped plates 410 are included, and the two arc-shaped plates 410 are arranged side by side and both extend along the axial direction of the handle 110. Each of the two first end surfaces of the first inner sliding member 120 is provided with a positioning groove 420, and each of the two second end surfaces of the second inner sliding member 130 is defined with a positioning groove 420. The arc-shaped plate 410 may be an arc-shaped plate structure, and is symmetrically arranged by the positioning grooves 420, so that the first inner sliding member 120 and the second inner sliding member 130 are balanced in force during movement.

The implant delivery system further includes a proximal connector 430 and a distal connector 440 arranged at respective ends of the handle, and the proximal connector 430 and the distal connector 440 are connected by the arc-shaped plate 410. The arc-shaped plate 410 has a third proximal end and a third distal end opposite to each other. The third proximal end of the arc-shaped plate 410 is connected to the proximal connector 430, and the third distal end of the arc-shaped plate 410 is connected to the distal connector 440. After the proximal connector 430 and the distal connector 440 are connected by the arc-shaped plate 410, the purpose of circumferential position limiting can be achieved. The proximal connector 430 is connected to the fourth proximal end of the inner tube 500. When the handle is rotated, the proximal connector 430 is likely to be rotated with the handle in the circumferential direction, thereby driving the inner tube 500 to rotate in the circumferential direction, which affects the accuracy in positioning the stent. In this embodiment, after the proximal connector 430 and the distal connector 440 are connected by the arc-shaped plate 410, the circumferential position limiting can be achieved, thereby solving the problem that the proximal connector 430 will rotate with the handle along the circumferential direction. In some embodiments, the number of the arc-shaped plates 410 may be two, and the concave surfaces of the two arc-shaped plates 410 face with each other and define a second cavity. In some embodiments, the proximal connector 430 defines a first blind hole therein, and the third proximal end of the arc-shaped plate 410 is installed in the first blind hole; the distal connector 440 defines therein a second blind hole (not shown in the figure), and the third distal end of the arc-shaped plate 410 is installed in the second blind hole. The shapes of the first blind hole and the second blind hole are adapted to the shape of the ends of the arc-shaped plate 410. In other words, when the arc-shaped plate 410 has an arc plate structure, the first blind hole and the second blind hole are arc-shaped holes. In this embodiment, the number of the first blind hole and the second blind hole are both two for installing the two arc-shaped plates 410. In some embodiments, the distal connector 440 further defines therein a first through hole, and the jacking tube 300 and the outer tube 200 pass through the first through hole.

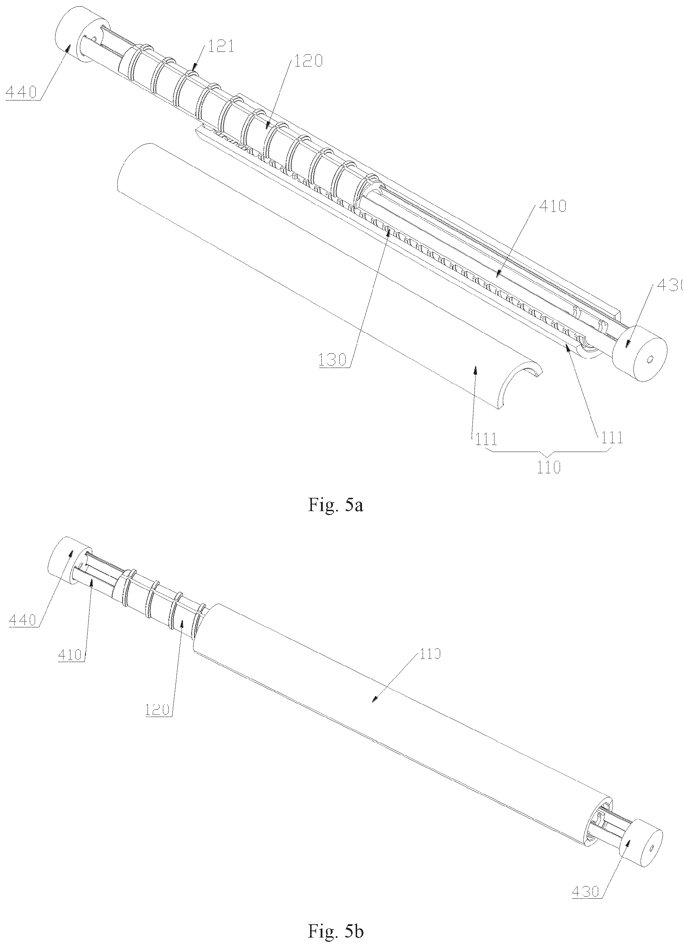

Further, the handle 110 may include a first cavity having a hollow tube-like shape, and the first inner sliding member 120 and the second inner sliding member 130 are arranged in the handle 110. The handle 110 is preferably composed of two parts. Please refer to Figs. 5a and 5b, the handle 110 includes two sub-handles 111. In a plane perpendicular to the axial direction of the handle 110, the inner cavity of each of the sub-handles 111 has a semicircular arc shape, and the radial dimensions of the two sub-handles 111 are completely the same. A first internal thread and a second internal thread are provided on the concave surface of each of the sub-handles 111. The two sub-handles 111 are connected to form a cavity penetrating along the axial direction of the handle 110 to accommodate the first inner sliding member 120 and the second inner sliding member 130. The first internal thread is matched with the first external thread 121 for screw transmission, and the second internal thread is matches with the second external thread 131 for screw transmission.

In addition, referring to Figs. 1 and 6, the implant delivery system provided by the embodiments of the present invention further includes an inner tube 500, a guide member 600, a first infusion tube 710 and a second infusion tube 810.

As shown in Fig. 6, the inner tube 500 is arranged in the jacking tube 300. In other words, the inner tube 500, the jacking tube 300, and the outer tube 200 are sleeved in the order from the inside to the outside. The inner tube 500 has a fourth proximal end and a fourth distal end opposite to each other. The fourth distal end of the inner tube 500 protrudes from the second distal end of the jacking tube 300 and extends inside the outer tube 200. An annular cavity is formed between the inner tube 500 and the outer tube 200, and the stent 10 can be compressed into the annular cavity. The fourth distal end of the inner tube 500 is connected to the guide member 600. The guide member 600 usually has a tapered structure, and the tip of the tapered structure is away from the fourth end of the inner tube. When the stent 10 is not released, the guiding member 600 abuts against the first distal end of the outer tube 200 with a smooth transition therebetween.

As shown in Fig. 7, the fourth proximal end of the inner tube 500 protrudes from the second proximal end of the jacking tube 300 and extends in the second inner sliding member 130. The fourth proximal end of the inner tube 500 is connected to the first infusion tube 710, and the end of the first infusion tube 710 away from the inner tube 500 can be provided with a luer taper 720 so as to be connected with an external syringe to discharge the air in the inner tube 500 during the use.

Furthermore, the fourth proximal end of the inner tube 500 can also be connected to the proximal connector 430. In some embodiments, the proximal connector 430 defines therein a second through hole penetrating in the axial direction, and the inner tube 500 extends through the first through hole, the second cavity, and the second through hole. The present invention does not particularly limit the connection mode of the inner tube 500 with the proximal connector 430, and the detachable mode is preferred, which improves the convenience in operation. In this way, when the stent 10 is completely released, the inner tube 500 and the proximal connector 430 can be disconnected.

In detail, the fourth proximal end of the inner tube 500 is provided with an inner tube connection assembly 510. The inner tube connection assembly 510 may include a first connecting sleeve 511, a second connecting sleeve 512, and a locking cap 513. The first connecting sleeve 511 is used to connect the fourth proximal end of the inner tube 500 and the first infusion tube 710, the second connecting sleeve 512 is sleeved over the first connecting sleeve 511, and the locking cap 513 is used to detachably connect the first connecting sleeve 511 and the second connecting sleeve 512. For example, the locking cap 513 is threadedly connected to the second connecting sleeve 512. When the locking cap 513 is tightened, the first connecting sleeve 511 and the second connecting sleeve 512 are tightly connected. When the locking cap 513 is loosen, the first connecting sleeve 511 and the first connecting sleeve 511 are relatively movable. The second connecting sleeve 512 is connected to the proximal connector 430. In this way, the position of the fourth proximal end of the inner tube 500 can be fixed by tightening the locking cap 513, which prevents the inner tube 500 from moving in the axial direction due to friction during the release of the stent 10 and causing displacement. When the stent 10 is released, the guide member 600 is separated from the outer tube 200, which causes an inconvenience in withdrawning the implant delivery system from the human body. However, after the stent 10 is released, the first connecting sleeve 511 and the second connecting sleeve 512 are disconnected, and the first infusion tube 710 can be pulled to make the inner tube 500 to move proximally so that the guide member 600 abuts aginst the outer tube 200 again, which facilitates withdrawal of the implant delivery system. It should be understood that the fourth proximal end of the inner tube 500 can also be connected to other elements such as an external mechanism to achieve the same purpose.

The fourth distal end of the inner tube 500 is further provided with a developing ring 520 to facilitate positioning of the stent 10. The number of the developing ring 520 may be one or more.

Please continue to refer to Figs. 6, 8 and 9, the second distal end of the jacking tube 300 is provided with a head 310, and the head 310 has a holding structure 311. The holding structure 311 is used for holding and releasing the stent 10. Specifically, the head 310 of the jacking tube is sleeved over the jacking tube 300, and the part of the jacking tube 300 away from the second distal end of the jacking tube 300 is preferably designed as an umbrella-shaped structure, and the outer surface of the umbrella-shaped structure is provided with the holding structure 311. Preferably, the holding structure 311 is a hook, the number of the hook is at least two, and the at least two of the hooks can be arranged at intervals along the circumferential direction of the head 310 of the jacking tube.

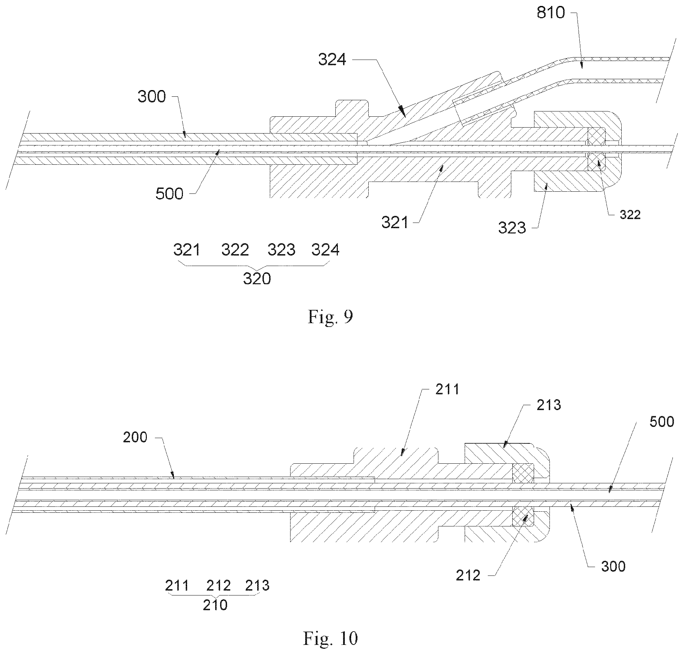

As shown in Fig. 9, the second proximal end of the jacking tube 300 is provided with a jacking tube connection assembly 320 used to connect the jacking tube 300 with the inner tube 500, and the jacking tube connection assembly 320 is also used to connect with the second inner sliding member 130.

The jacking tube connection assembly 320 includes a jacking tube connector 321 arranged at the second proximal end of the jacking tube 300, and a second connecting hole is defined in the jacking tube connector 321, and the jacking tube connector is sleeved over the the inner tube 500 through the second connecting hole and is slidable along the inner tube 500. The jacking tube connector 321 is in sealing connection with the inner tube 500. To this end, the jacking tube connection assembly 320 further includes a second sealing ring 322 and a second sealing cap 323, and the second sealing ring 322 is sleeved over the inner tube 500 and fastened by the second sealing cap 323 so that it is in close contact with the jacking tube connector 321 to achieve the purpose of sealing.

The jacking tube connector 321 may further be defined with a second snap groove. Correspondingly, a second snap ring 133 can be provided on the inner wall of the second inner sliding member 130. The jacking tube 300 can be detachably connected to the second inner sliding member 130 by means of the fit between the second snap groove and the second snap ring 133.

Referring to Fig. 10, the first proximal end of the outer tube 200 is provided with an outer tube connection assembly 210. The outer tube 200 is connected to the jacking tube 300 at the outside of the jacking tube 300 by the outer tube connection assembly 210 and is slidable along the jacking tube 300, and the outer tube connection assembly shall be in sealing connection with the jacking tube 300. Specifically, the outer tube connection assembly 210 includes an outer tube connector 211, a first sealing ring 212, and a second sealing cap 213. A first connecting hole is defined in the tube connector 211. The first connecting hole has an end fixedly connected to the outer tube 200, and a further end sleeved over the jacking tube 300. The first sealing ring 212 is sleeved over the jacking tube 300 and is in tight contact with the outer tube 200 by means of the first sealing cap 213 for sealing.

The outer tube connector 211 may further be defined with a first snap groove, and the first inner sliding member 120 may be correspondingly provided with a first snap ring 123. The outer tube 200 can be detachably connected to the first inner sliding member 120 by means of the fit between the first snap groove and the first snap ring 123.

In addition, the second infusion tube 810 is used to discharge the air between the outer tube 200 and the jacking tube 300 and the air between the jacking tube 300 and the inner tube 500. In detail, the arrangement of the second infusion tube may be selected from, but not limited to, the following two options:
The first arrangement (not shown): the outer tube connector is provided with a first branch connector in wich a third connecting hole is defined. The second infusion tube is inserted in the third connecting hole. The end of the second infusion tube away from the first branch connector is provided with a check valve.

The second arrangement: as shown in Fig. 9, the jacking tube connection assembly 320 further includes a second branch connector 324 in which a fourth connecting hole is defined. The second infusion tube 810 is inserted into the fourth connecting hole so that the jacking tube 300 is in communication with the second infusion tube 810. The end of the second infusion tube 810 away from the jacking tube connector 321 is provided with a check valve 820. It should be noted that various existing methods can be used to realize the fixed connection between the components when assembling the components. For example, the guide member 600 may be injection molded on the fourth distal end of the inner tube 500, and the head 310 of the jacking tube may be injection molded on or in hot melt connection with the second distal end of the jacking tube 300, and the outer tube connector 211 may be injection molded on the first proximal end of the outer tube 200, the inner tube 500 and the first connecting sleeve 511 may be bonded by glue, the second infusion tube 810 and the second branch connector 324 may be bonded by glue.

The method of using the implant delivery system of the present invention will be described in the following by taking the delivery of the stent 10 into the diseased blood vessel as an example.

Before the surgical operation begins, the stent 10 is first compressed into the outer tube 200; then a syringe is connected to the check valve 820, and a liquid such as heparin saline is injected into the implant delivery system by the syringe to discharge the air between the inner tube 500 and the jacking tube 300 as well as the air between the jacking tube 300 and the outer tube 200; and a syringe is connected to the Luer taper 720 to inject liquid into the inner tube 500 to discharge the air in the inner tube 500; the outer wall of the outer tube 200 is then cleaned by heparin saline.

After the surgical operation begins, the blood vessel is first punctured, and then under the supervision of the X-ray machine, the guide member 600 is sent to the area where the lesion locates by using a common means (such as a guide wire) to make the developing ring 520 coincide in position with the location where and the stent 10 is to be placed. Then the handle 110 is rotated to move the outer tube 200 proximally, and the stent 10 is released and expanded. At the same time, the jacking tube 300 pushes the stent 10 to move distally so that the stent 10 is finally located at a predetermined position, and the guide member 600 is separated from the outer tube 200; the handle 110 is continuously rotated until the stent 10 is completely released. Subsequently, the locking cap 513 is released, and the first infusion tube 710 is pulled proximally to drive the inner tube 500 to move proximally until the guide member 600 contacts the outer tube 200 again, and finally the implant delivery system is withdrawn from the body.

In the process of releasing the stent 10, if the operator is not satisfied with the position of the stent 10, before the stent 10 is completely released, the handle 110 can be reversely rotated to move the outer tube 200 toward the distal end, while the jacking tube 300 is retracted to pull the stent 10 back into the outer tube 200; then, the operator readjusts the position of the stent 10 and releases it.

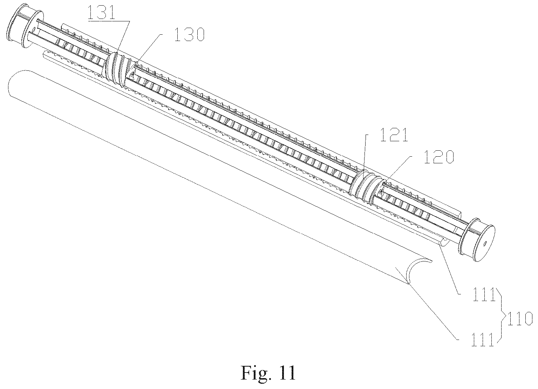

In the above embodiments, the first inner sliding member 120 and the second inner sliding member 130 are arc-shaped when viewed along the axial direction of the handle 110, which is taken as an example to describe the structure of the implant delivery system. However, structures of the first inner sliding member 120 and the second inner sliding member 130 are not limited thereto. For example, in another embodiment, as shown in Fig. 11, the handle 110 has a first cavity extending axially with a cylinder shape, and the wall of the first cavity is formed with a first internal thread and a second internal thread, and the first internal thread and the second internal thread are arranged along the axial direction of the handle 110. The first inner sliding member 120 has a hollow tube-shaped structure with a first external thread 121 provided on its outer surface, and the first inner sliding member 120 is disposed in the first cavity of the handle 110, and the first external thread 121 is matched with the first internal thread. The second inner sliding member 130 has a hollow tube-shaped structure with a second external thread 131 disposed on its outer surface, and the second inner sliding member 130 is disposed in the first cavity of the handle 110 and is arranged coaxially with the first inner sliding member 120, and the second external thread 131 is threadedly matched with the second internal thread.

In short, the present invention does not strictly limit the specific structures of the first inner sliding member, the second inner sliding member, and the handle, as long as the first inner sliding member and the second inner sliding member can simultaneously move linearly in opposite directions in response to the rotation of the handle after they are threadedly connected to the handle.

The advantage of the implant delivery system provided in the embodiments of the present invention is as follows. On one hand, the outer tube 200 and the jacking tube 300 are simultaneously driven by the driving assembly 100 to move linearly in opposite directions, so that the stent 10 gradually moves from the distal end to the proximal end, and the stent 10 is pushed by the jacking tube 300 to move distally to supplement the displacement caused by the contraction of the stent 10 due to expansion, so that the stent 10 can be delivered to a predetermined position to ensure the treatment effect. On the other hand, the outer tube 200 and the jacking tube 300 are driven to move by means of screw feeding, which facilitates the control of the moving distance of the outer tube 200 and the jacking tube 300, so as to achieve accurat positioning of the stent 10 and reduced requirement for the operator's experience.

The implant delivery system provided in the embodiments of the present invention has further advantage as follows. The proximal connector 430 and the distal connector 440 are connected by the arc-shaped plate 410, which can achieve the purpose of circumferential position limiting, thereby avoiding the situation that inner tube 500 is driven by the proximal connector 430 to rotate along the circumferential direction with the handle, which affects the accuracy in positioning the stent.

Although the present invention is disclosed as above, it is not limited thereto. It is apparent that those skilled in the art can make various modifications and variations to the present invention without departing from the scope thereof. Accordingly, the invention is intended to embrace all such modifications and variations if they fall within the scope of the appended claims.

## Claims

1. An implant (10) delivery system, comprising: a handle (110), a first inner sliding member (120), a second inner sliding member (130), an inner tube (500), an outer tube (200), and a jacking tube (300), wherein the handle (110) has a first cavity in which the first inner sliding member (120) and the second inner sliding member (130) are located;
the outer tube (200) has a first proximal end and a first distal end opposite to each other, and the first proximal end is connected to the first inner sliding member (120); the jacking tube (300) has a second proximal end and a second distal end opposite to each other, the second distal end is arranged in the outer tube (200), and the second proximal end is connected to the second inner sliding member (130); the inner tube (500) extends through the outer tube (200) and the jacking tube (300), and a cavity configured to receive a implant (10) is formed between the inner tube (500) and the outer tube (200); and the second distal end is configured for connecting with a proximal part of the implant (10);
the handle (110) is threadedly matched with the first inner sliding member and the second inner sliding member (130) for screw transmission, and configured to drive the first inner sliding member (120) and the second inner sliding member (130) to move linearly along an axis of the handle (110) in opposite directions so as to drive the outer tube (200) and the jacking tube (300) to move in the opposite directions to release the implant (10) from a distal end to a proximal end thereof;
wherein the implant (10) delivery system further comprises a proximal connector (430) and a distal connector (440) provided at respective ends of the handle (110), the proximal connector (430) and the distal connector (440) are connected by two arc-shaped plates (410) having concave surfaces which are arranged opposite to each other and define a second cavity; the distal connector (440) is defined with a first through hole penetrating in an axial direction; the proximal connector (430) is connected to a proximal end of the inner tube (500);
wherein a positioning groove (420) extending along an axial direction of the handle (110) is defined in each of the first inner sliding member (120) and the second inner sliding member (130), and the first inner sliding member (120) and the second inner sliding member (130) are slidably connected to an arc-shaped plate (410) through the positioning groove (420); the jacking tube (300) passes through the first through hole.

2. The implant (10) delivery system according to claim 1, wherein an outer surface of the first inner sliding member (120) is provided with a first external thread (121), and an outer surface of the second inner sliding member (130) is provided with a second external thread (131), thread directions of the first external thread (121) and the second external thread (131) are opposite;
wherein an inner surface of the handle (110) is provided with a first internal thread and a second internal thread, and the first internal thread of the handle (110) is threadedly matched with the first external thread (121) of the first inner sliding member (120) for screw transmission, and the second internal thread of the handle (110) is threadedly matched with the second external thread (131) of the second inner sliding member (130) for screw transmission.

3. The implant (10) delivery system according to claim 1, wherein the handle (110) comprises two sub-handles (111) having inner cavities, and each of the inner cavities has a semicircular arc shape on a cross section perpendicular to an axial direction of the handle (110).

4. The implant (10) delivery system according to any one of claims 1 to 3, wherein each of the first inner sliding member (120) and the second inner sliding member (130) has an arc shape, preferably a semicircular arc shape, on a cross section perpendicular to the axis of the handle (110), and a concave surface of the first inner sliding member (120) is arranged opposite to a concave surface of the second inner sliding member (130).

5. The implant (10) delivery system of claim 1, wherein the first inner sliding member (120) has two first end surfaces extending along the axial direction of the handle (110), the positioning groove (420) is defined in each of the two first end surfaces, and the first inner sliding member (120) is slidably connected to the two arc-shaped plates (410) through the two positioning grooves (420); the second inner sliding member (130) has two second end surfaces extending along the axial direction of the handle (110), the positioning groove (420) is further defined in each of the two second end surfaces, and the second inner sliding member (130) is slidably connected to the two arc-shaped plates (410) through the two positioning grooves (420);
wherein the first end surface is provided with a raised limiting member (122), the second end surface is defined with a sliding groove (132) extending along the axial direction of the handle (110), and the limiting member is installed in the sliding groove (132); or, the first end surface is defined with a sliding groove, the second end surface is provided with a raised limiting member (122) extending along the axial direction of the handle (110), and the limiting member is installed in the sliding groove (132).

6. The implant (10) delivery system according to claim 1, wherein the proximal connector (430) is defined with a second through hole penetrating in the axial direction, and the inner tube (500) extends through the first through hole, the second cavity and the second through hole.

7. The implant (10) delivery system according to claim 1, wherein the arc-shaped plate (410) has a third proximal end and a third distal end opposite to each other; a first blind hole is defined in the proximal connector (430), and the third proximal end of the arc-shaped plate (410) is installed in the first blind hole; a second blind hole is defined in the distal connector (440), and the third distal end of the arc-shaped plate (410) is installed in the second blind hole.

8. The implant (10) delivery system according to claim 1, wherein further comprising a head of the jacking tube (310) arranged at the second distal end of the jacking tube (300), and the head is provided with a holding structure for holding and releasing the implant (10);
wherein the holding structure comprises a plurality of hooks (311) spaced apart along a circumferential direction of the head of the jacking tube (310).

9. The implant (10) delivery system according to claim 1, wherein further comprising a guide member (600), and the inner tube (500) has a fourth proximal end and a fourth distal end opposite to each other, the fourth distal end of the inner tube (500) protrudes from the first distal end of the outer tube (200) and is connected to the guide member (600);
wherein further comprising at least a developing ring (520) arranged at the fourth distal end of the inner tube (500).

10. The implant (10) delivery system according to claim 1, wherein further comprising a guide member (600), and the inner tube (500) has a fourth proximal end and a fourth distal end opposite to each other, the fourth distal end of the inner tube (500) protrudes from the first distal end of the outer tube (200) and is connected to the guide member (600);
wherein the first proximal end of the outer tube (200) is connected to an outer wall of the jacking tube (300) through an outer tube connection assembly (210), and the outer tube (200) is slidable along the outer wall of the jacking tube (300);
wherein the outer tube connection assembly (210) comprises an outer tube connector (211), a first sealing ring (212) and a first sealing cap (213); the outer tube connector (211) has a first connection hole whose one end is fixedly connected to the outer tube (200), a further end is sleeved over the jacking tube (300); and the first sealing ring (212) is sleeved over the jacking tube (300) and is in sealing connection with the outer tube (200) when the first sealing cap (213) is tightened.

11. The implant (10) delivery system according to claim 10, wherein the outer tube connector (211) is further defined with a first snap groove, the first inner sliding member (120) is provided with a first snap ring (123), and the first snap groove is detachably connected to the first snap ring (123).

12. The implant (10) delivery system according to claim 10, wherein the second proximal end of the jacking tube (300) is provided with a jacking tube connection assembly (320) comprising a jacking tube connector (321), wherein the jacking tube connector (321) is arranged at the second proximal end of the jacking tube (300), and the jacking tube connector (321) is defined with a second connecting hole through which the jacking tube connector (321) is sleeved over the inner tube (500) and is slidable along the inner tube (500);
wherein the jacking tube connection assembly further comprises a second sealing ring (322) and a second sealing cap (323), the second sealing ring (322) is sleeved over the inner tube (500), the second sealing cap (323) is used to fasten the second sealing ring (322) so that the jacking tube connector (321) is in sealing connection with the inner tube (500).

13. The implant (10) delivery system according to claim 10, wherein the second proximal end of the jacking tube (300) is provided with a jacking tube connection assembly (320) comprising a jacking tube connector (321), wherein the jacking tube connector (321) is arranged at the second proximal end of the jacking tube (300), and the jacking tube connector (321) is defined with a second connecting hole through which the jacking tube connector (321) is sleeved over the inner tube (500) and is slidable along the inner tube (500);
wherein the jacking tube connector (321) is defined with a second snap groove, the second inner sliding member (130) is provided with a second snap ring (133), and the second snap groove is detachably connected to the second snap ring (133).

14. The implant (10) delivery system according to claim 12, wherein comprising a first infusion tube (710) and a second infusion tube (810); the fourth proximal end of the inner tube (500) protrudes from the second proximal end of the jacking tube (300) and is in communication with the first infusion tube (710); the outer tube connector (211) is provided with a first branch connector, and a third connecting hole is defined in the first branch connector and is in communication with the second infusion tube (810); or,
the tube jacking tube connector (321) is provided with a second branch connector (324), and a fourth connecting hole is defined in the second branch connector (324) and is in communication with the second infusion tube (810);
wherein the fourth proximal end of the inner tube (500) is provided with an inner tube connection assembly (510) comprising a first connecting sleeve (511), a second connecting sleeve (512) and a locking cap (513); the first connecting sleeve (511) is used to connect the inner tube (500) and the first infusion tube (710), the second connecting sleeve (512) is sleeved over the first connecting sleeve (511) and is used to fix the fourth proximal end of the inner tube (500), and the locking cap (513) is used to lock the second connecting sleeve (512), the first connecting sleeve (511) and the first infusion tube (710) in position.

15. The implant (10) delivery system according to any one of claims 1 to 3, wherein the first inner sliding member (120) and the second inner sliding member (130) are shaped as two hollow tube-shaped structures arranged coaxially with each other.

## Patentansprüche

1. Einführungssystem für ein Implantat (10), umfassend: einen Handgriff (110), ein erstes inneres Gleitelement (120), ein zweites inneres Gleitelement (130), ein inneres Rohr (500), ein äußeres Rohr (200) und ein Vorschubrohr (300), wobei der Handgriff (110) einen ersten Hohlraum aufweist, in dem sich das erste innere Gleitelement (120) und das zweite innere Gleitelement (130) befinden;
das äußere Rohr (200) ein erstes proximales Ende und ein erstes distales Ende aufweist, die einander gegenüberliegen, und das erste proximale Ende mit dem ersten inneren Gleitelement (120) verbunden ist; das Vorschubrohr (300) ein zweites proximales Ende und ein zweites distales Ende aufweist, die einander gegenüberliegen, das zweite distale Ende in dem äußeren Rohr (200) angeordnet ist, und das zweite proximale Ende mit dem zweiten inneren Gleitstück (130) verbunden ist; das innere Rohr (500) sich durch das äußere Rohr (200) und das Vorschubrohr (300) erstreckt und ein Hohlraum, der zum Aufnehmen eines Implantats (10) konfiguriert ist, zwischen dem inneren Rohr (500) und dem äußeren Rohr (200) gebildet ist; und das zweite distale Ende zum Verbinden mit einem proximalen Teil des Implantats (10) konfiguriert ist;
der Handgriff (110) mit dem ersten inneren Gleitelement und dem zweiten inneren Gleitelement (130) zur Schraubenübertragung verschraubt ist und konfiguriert ist, um das erste innere Gleitelement (120) und das zweite innere Gleitelement (130) anzutreiben, um sich linear entlang einer Achse des Handgriffs (110) in entgegengesetzte Richtungen zu bewegen, um das äußere Rohr (200) und das Vorschubrohr (300) anzutreiben, um sich in die entgegengesetzten Richtungen zu bewegen, um das Implantat (10) von einem distalen Ende zu einem proximalen Ende davon zu lösen;
wobei das Einführungssystem für ein Implantat (10) ferner ein proximales Verbinder (430) und ein distales Verbinder (440) umfasst, die an den jeweiligen Enden des Handgriffs (110) bereitgestellt sind, der proximale Verbinder (430) und der distale Verbinder (440) durch zwei bogenförmige Platten (410) mit konkaven Oberflächen verbunden sind, die einander gegenüberliegend angeordnet sind und einen zweiten Hohlraum definieren; der distale Verbinder (440) mit einem ersten Durchgangsloch versehen ist, das in axialer Richtung durchdringt; der proximale Verbinder (430) mit einem proximalen Ende des inneren Rohrs (500) verbunden ist;
wobei eine Stellnut (420), die sich entlang einer axialen Richtung des Handgriffs (110) erstreckt, sowohl in dem ersten inneren Gleitelement (120) als auch in dem zweiten inneren Gleitelement (130) definiert ist, und das erste innere Gleitelement (120) und das zweite innere Gleitelement (130) durch die Stellnut (420) gleitend mit einer bogenförmigen Platte (410) verbunden sind; das Vorschubrohr (300) durch das erste Durchgangsloch verläuft.

2. Einführungssystem für ein Implantat (10) nach Anspruch 1, wobei eine Außenfläche des ersten inneren Gleitelements (120) mit einem ersten Außengewinde (121) versehen ist, und eine Außenfläche des zweiten inneren Gleitelements (130) mit einem zweiten Außengewinde (131) versehen ist, wobei die Gewinderichtungen des ersten Außengewindes (121) und des zweiten Außengewindes (131) entgegengesetzt sind;
wobei eine Innenfläche des Handgriffs (110) mit einem ersten Innengewinde und einem zweiten Innengewinde versehen ist und das erste Innengewinde des Handgriffs (110) mit dem ersten Außengewinde (121) des ersten inneren Gleitelements (120) zur Schraubübertragung verschraubt ist, und das zweite Innengewinde des Handgriffs (110) mit dem zweiten Außengewinde (131) des zweiten inneren Gleitelements (130) zur Schraubübertragung verschraubt ist.

3. Einführungssystem für ein Implantat (10) nach Anspruch 1, wobei der Handgriff (110) zwei Teilhandgriffe (111) umfasst, die innere Hohlräume aufweisen, und jeder der inneren Hohlräume eine halbkreisförmige Bogenform in einem Querschnitt senkrecht zu einer axialen Richtung des Handgriffs (110) aufweist.

4. Einführungssystem für ein Implantat (10) nach einem der Ansprüche 1 bis 3, wobei sowohl das erste innere Gleitelement (120) als auch das zweite innere Gleitelement (130) eine Bogenform, vorzugsweise eine halbkreisförmige Bogenform, in einem Querschnitt senkrecht zu der Achse des Handgriffs (110) aufweist, und eine konkave Fläche des ersten inneren Gleitelements (120) gegenüber einer konkaven Fläche des zweiten inneren Gleitelements (130) angeordnet ist.

5. Einführungssystem für ein Implantat (10) nach Anspruch 1, wobei das erste innere Gleitelement (120) zwei erste Endflächen aufweist, die sich entlang der axialen Richtung des Handgriffs (110) erstrecken, die Stellnut (420) in jeder der zwei ersten Endflächen definiert ist, und das erste innere Gleitelement (120) durch die zwei Stellnuten (420) gleitend mit den zwei bogenförmigen Platten (410) verbunden ist; das zweite innere Gleitelement (130) zwei zweite Endflächen aufweist, die sich entlang der axialen Richtung des Handgriffs (110) erstrecken, die Stellnut (420) ferner in jeder der zwei zweiten Endflächen definiert ist, und das zweite innere Gleitelement (130) durch die zwei Stellnuten (420) gleitend mit den zwei bogenförmigen Platten (410) verbunden ist;
wobei die erste Endfläche mit einem erhabenen Begrenzungselement (122) versehen ist, die zweite Endfläche mit einer sich entlang der axialen Richtung des Handgriffs (110) erstreckenden Gleitnut (132) definiert ist und das Begrenzungselement in der Gleitnut (132) installiert ist; oder, die erste Endfläche mit einer Gleitnut definiert ist, die zweite Endfläche mit einem erhöhten Begrenzungselement (122) versehen ist, das sich entlang der axialen Richtung des Handgriffs (110) erstreckt, und das Begrenzungselement in der Gleitnut (132) installiert ist.

6. Einführungssystem für ein Implantat (10) nach Anspruch 1, wobei der proximale Verbinder (430) mit einem zweiten Durchgangsloch definiert ist, das in der axialen Richtung durchdringt, und das innere Rohr (500) sich durch das erste Durchgangsloch, den zweiten Hohlraum und das zweite Durchgangsloch erstreckt.

7. Einführungssystem für ein Implantat (10) nach Anspruch 1, wobei die bogenförmige Platte (410) ein drittes proximales Ende und ein drittes distales Ende aufweist, die einander gegenüberliegen; ein erstes Sackloch in dem proximalen Verbinder (430) definiert ist, und das dritte proximale Ende der bogenförmigen Platte (410) in dem ersten Sackloch installiert ist; ein zweites Sackloch in dem distalen Verbinder (440) definiert ist und das dritte distale Ende der bogenförmigen Platte (410) in dem zweiten Sackloch installiert ist.

8. Einführungssystem für ein Implantat (10) nach Anspruch 1, wobei ferner umfassend einen Kopf des Vorschubrohrs (310), der an dem zweiten distalen Ende des Vorschubrohrs (300) angeordnet ist, und der Kopf mit einer Haltestruktur zum Halten und Freigeben des Implantats (10) versehen ist;
wobei die Haltestruktur eine Vielzahl von Haken (311) umfasst, die entlang einer Umfangsrichtung des Kopfs des Vorschubrohrs (310) beabstandet sind.

9. Einführungssystem für ein Implantat (10) nach Anspruch 1, wobei ferner umfassend ein Führungselement (600) und das innere Rohr (500) ein viertes proximales Ende und ein viertes distales Ende aufweist, die einander gegenüberliegen, das vierte distale Ende des inneren Rohrs (500) über das erste distale Ende des äußeren Rohrs (200) hervorsteht und mit dem Führungselement (600) verbunden ist;
wobei ferner umfassend mindestens ein Entwicklungsring (520), das an dem vierten distalen Ende des inneren Rohrs (500) angeordnet ist.

10. Einführungssystem für ein Implantat (10) nach Anspruch 1, wobei ferner umfassend ein Führungselement (600) und das innere Rohr (500) ein viertes proximales Ende und ein viertes distales Ende aufweist, die einander gegenüberliegen, das vierte distale Ende des inneren Rohrs (500) über das erste distale Ende des äußeren Rohrs (200) hervorsteht und mit dem Führungselement (600) verbunden ist;
wobei das erste proximale Ende des äußeren Rohrs (200) mit einer Außenwand des Vorschubrohrs (300) durch eine äußere Rohrverbindungsanordnung (210) verbunden ist und das äußere Rohr (200) entlang der Außenwand des Vorschubrohrs (300) verschiebbar ist;
wobei die äußere Rohrverbindungsanordnung (210) einen äußeren Rohrverbinder (211), einen ersten Dichtungsring (212) und eine erste Dichtungskappe (213) umfasst; der äußeren Rohrverbinder (211) ein erstes Verbindungsloch aufweist, dessen eine Ende fest mit dem äußeren Rohr (200) verbunden ist, ein weiteres Ende über das Vorschubrohr (300) aufgesteckt ist; und der erste Dichtungsring (212) über das Vorschubrohr (300) aufgesteckt ist und in abdichtender Verbindung mit dem äußeren Rohr (200) ist, wenn die erste Dichtungskappe (213) angezogen ist.

11. Einführungssystem für ein Implantat (10) nach Anspruch 10, wobei der äußere Rohrverbinder (211) ferner mit einer ersten Rastnut versehen ist, das erste innere Gleitelement (120) mit einem ersten Einrastring (123) versehen ist, und die erste Rastnut lösbar mit dem ersten Einrastring (123) verbunden ist.

12. Einführungssystem für ein Implantat (10) nach Anspruch 10, wobei das zweite proximale Ende des Vorschubrohrs (300) mit einer Vorschubrohrverbindungsanordnung (320) versehen ist, umfassend einen Vorschubrohrverbinder (321), wobei das Vorschubrohrverbinder (321) an dem zweiten proximalen Ende des Vorschubrohrs (300) angeordnet ist und das Vorschubrohrverbinder (321) mit einem zweiten Verbindungsloch definiert ist, durch das das Vorschubrohrverbinder (321) über das innere Rohr (500) geschoben wird und entlang des inneren Rohrs (500) verschiebbar ist;
wobei die Verbindungsanordnung für das Vorschubrohr ferner einen zweiten Dichtungsring (322) und eine zweite Dichtungskappe (323) umfasst, der zweite Dichtungsring (322) über das innere Rohr (500) aufgesteckt wird, die zweite Dichtungskappe (323) zur Befestigung des zweiten Dichtungsrings (322) verwendet wird, sodass der Vorschubrohranschluss (321) in dichtender Verbindung mit dem inneren Rohr (500) ist.

13. Einführungssystem für ein Implantat (10) nach Anspruch 10, wobei das zweite proximale Ende des Vorschubrohrs (300) mit einer Vorschubrohrverbindungsanordnung (320) versehen ist, umfassend einen Vorschubrohrverbinder (321), wobei das Vorschubrohrverbinder (321) an dem zweiten proximalen Ende des Vorschubrohrs (300) angeordnet ist und das Vorschubrohrverbinder (321) mit einem zweiten Verbindungsloch definiert ist, durch das der Vorschubrohrverbinder (321) über das innere Rohr (500) geschoben wird und entlang des inneren Rohrs (500) verschiebbar ist;
wobei der Vorschubrohrverbinder (321) mit einer zweiten Rastnut versehen ist, das zweite innere Gleitelement (130) mit einem zweiten Einrastring (133) versehen ist und die zweite Rastnut lösbar mit dem zweiten Einrastring (133) verbunden ist.

14. Einführungssystem für ein Implantat (10) nach Anspruch 12, wobei umfassend ein erstes Infusionsrohr (710) und ein zweites Infusionsrohr (810); wobei das vierte proximale Ende des inneren Rohrs (500) aus dem zweiten proximalen Ende des Vorschubrohrs (300) hervorsteht und mit dem ersten Infusionsrohr (710) in Verbindung ist; der äußere Rohrverbinder (211) mit einem ersten Verzweigungsverbinder versehen ist und ein drittes Verbindungsloch in dem ersten Abzweigverbinder definiert ist und in Verbindung mit dem zweiten Infusionsrohr (810) ist; oder,
der Vorschubrohrverbinder (321) mit einem zweiten Abzweigverbinder (324) versehen ist, und ein viertes Verbindungsloch in dem zweiten Abzweigverbinder (324) definiert ist und mit dem zweiten Infusionsrohr (810) in Verbindung ist;
wobei das vierte proximale Ende des inneren Rohrs (500) mit einer innere Rohrverbindungsanordnung (510) versehen ist, die eine erste Verbindungsmuffe (511), eine zweite Verbindungsmuffe (512) und eine Verschlusskappe (513) umfasst; die erste Verbindungshülse (511) verwendet wird, um das innere Rohr (500) und das erste Infusionsrohr (710) zu verbinden, die zweite Verbindungshülse (512) auf die die erste Verbindungshülse (511) aufgesteckt und verwendet wird, um das vierte proximale Ende des inneren Rohrs (500) zu fixieren, und die Verriegelungskappe (513) verwendet wird, um die zweite Verbindungshülse (512), die erste Verbindungshülse (511) und das erste Infusionsrohr (710) in ihrer Position zu verriegeln.

15. Einführungssystem für ein Implantat (10) nach einem der Ansprüche 1 bis 3, wobei das erste innere Gleitelement (120) und das zweite innere Gleitelement (130) als zwei koaxial zueinander angeordnete hohle röhrenförmige Strukturen gebildet sind.

## Revendications

1. Système de pose d'implant (10) comprenant : une poignée (110), un premier élément coulissant interne (120), un second élément coulissant interne (130), un tube interne (500), un tube externe (200) et un tube de poussée (300), dans lequel la poignée (110) présente une première cavité dans laquelle se trouvent le premier élément coulissant interne (120) et le second élément coulissant interne (130) ;
le tube externe (200) a une première extrémité proximale et une première extrémité distale opposées l'une à l'autre, et la première extrémité proximale est reliée au premier élément coulissant interne (120) ; le tube de poussée (300) a une seconde extrémité proximale et une seconde extrémité distale opposées l'une à l'autre, la seconde extrémité distale est disposée dans le tube externe (200), et la seconde extrémité proximale est reliée au second élément coulissant interne (130) ; le tube interne (500) s'étend à travers le tube externe (200) et le tube de poussée (300), et une cavité configurée pour recevoir un implant (10) est formée entre le tube interne (500) et le tube externe (200) ; et la seconde extrémité distale est configurée pour se connecter à une partie proximale de l'implant (10) ;
la poignée (110) est en ajustement par filetage avec le premier élément coulissant interne et le second élément coulissant interne (130) pour une transmission à vis, et configurée pour entraîner le premier élément coulissant interne (120) et le second élément coulissant interne (130) à se déplacer linéairement le long d'un axe de la poignée (110) dans des directions opposées de manière à entraîner le tube externe (200) et le tube de poussée (300) à se déplacer dans des directions opposées pour libérer l'implant (10) d'une extrémité distale à une extrémité proximale ;
dans lequel le système de pose d'implant (10) comprend en outre un connecteur proximal (430) et un connecteur distal (440) situés aux extrémités respectives de la poignée (110), le connecteur proximal (430) et le connecteur distal (440) sont reliés par deux plaques en forme d'arc (410) présentant des surfaces concaves qui sont disposées à l'opposé l'une de l'autre et définissent une seconde cavité ; le connecteur distal (440) est défini par un premier trou traversant pénétrant dans une direction axiale ; le connecteur proximal (430) est relié à une extrémité proximale du tube interne (500) ;
dans lequel une rainure de positionnement (420) s'étendant le long d'une direction axiale de la poignée (110) est définie dans chacun du premier élément coulissant interne (120) et du second élément coulissant interne (130), et le premier élément coulissant interne (120) et le second élément coulissant interne (130) sont connectés de manière coulissante à une plaque en forme d'arc (410) à travers la rainure de positionnement (420) ; le tube de poussée (300) passe à travers le premier trou traversant.

2. Système de pose d'implant (10) selon la revendication 1, dans lequel une surface externe du premier élément coulissant interne (120) est pourvue d'un premier filetage externe (121), et une surface externe du second élément coulissant interne (130) est pourvue d'un second filetage externe (131), les directions de filetage du premier filetage externe (121) et du second filetage externe (131) sont opposées ;
dans lequel une surface interne de la poignée (110) est pourvue d'un premier filetage interne et d'un second filetage interne, et le premier filetage interne de la poignée (110) est en ajustement par filetage avec le premier filetage externe (121) du premier élément coulissant interne (120) pour une transmission à vis, et le second filetage interne de la poignée (110) est en ajustement par filetage avec le second filetage externe (131) du second élément coulissant interne (130) pour une transmission à vis.

3. Système de pose d'implant (10) selon la revendication 1, dans lequel la poignée (110) comprend deux sous-poignées (111) ayant des cavités internes, et chacune des cavités internes a une forme d'arc semi-circulaire sur une section transversale perpendiculaire à une direction axiale de la poignée (110).

4. Système de pose d'implant (10) selon l'une quelconque des revendications 1 à 3, dans lequel chacun du premier élément coulissant interne (120) et du second élément coulissant interne (130) a une forme d'arc, de préférence une forme d'arc semi-circulaire, sur une section transversale perpendiculaire à l'axe de la poignée (110), et une surface concave du premier élément coulissant interne (120) est disposée à l'opposé d'une surface concave du second élément coulissant interne (130).

5. Système de pose d'implant (10) selon la revendication 1, dans lequel le premier élément coulissant interne (120) a deux premières surfaces d'extrémité s'étendant le long de la direction axiale de la poignée (110), la rainure de positionnement (420) est définie dans chacune des deux premières surfaces d'extrémité, et le premier élément coulissant interne (120) est relié de manière coulissante aux deux plaques en forme d'arc (410) par l'intermédiaire des deux rainures de positionnement (420) ; le second élément coulissant interne (130) a deux secondes surfaces d'extrémité s'étendant le long de la direction axiale de la poignée (110), la rainure de positionnement (420) est en outre définie dans chacune des deux secondes surfaces d'extrémité, et le second élément coulissant interne (130) est relié de manière coulissante aux deux plaques en forme d'arc (410) par l'intermédiaire des deux rainures de positionnement (420) ;
dans lequel la première surface d'extrémité est pourvue d'un élément de limitation surélevé (122), la seconde surface d'extrémité est définie avec une rainure de coulissement (132) s'étendant le long de la direction axiale de la poignée (110), et l'élément de limitation est installé dans la rainure de coulissement (132) ; ou, la première surface d'extrémité est définie avec une rainure de coulissement, la seconde surface d'extrémité est pourvue d'un élément de limitation surélevé (122) s'étendant le long de la direction axiale de la poignée (110), et l'élément de limitation est installé dans la rainure de coulissement (132).

6. Système de pose d'implant (10) selon la revendication 1, dans lequel le connecteur proximal (430) est défini avec un deuxième trou traversant pénétrant dans la direction axiale, et le tube interne (500) s'étend à travers le premier trou traversant, la seconde cavité et le deuxième trou traversant.

7. Système de pose d'implant (10) selon la revendication 1, dans lequel la plaque en forme d'arc (410) a une troisième extrémité proximale et une troisième extrémité distale opposées l'une à l'autre ; un premier trou borgne est défini dans le connecteur proximal (430), et la troisième extrémité proximale de la plaque en forme d'arc (410) est installée dans le premier trou borgne ; un second trou borgne est défini dans le connecteur distal (440), et la troisième extrémité distale de la plaque en forme d'arc (410) est installée dans le second trou borgne.

8. Système de pose d'implant (10) selon la revendication 1, comprenant en outre une tête du tube de poussée (310) disposée à la seconde extrémité distale du tube de poussée (300), et la tête est pourvue d'une structure de maintien pour maintenir et libérer l'implant (10) ;
dans lequel la structure de maintien comprend une pluralité de crochets (311) espacés le long d'une direction circonférentielle de la tête du tube de poussée (310).

9. Système de pose d'implant (10) selon la revendication 1, comprenant en outre un élément de guidage (600), et le tube interne (500) a une quatrième extrémité proximale et une quatrième extrémité distale opposées l'une à l'autre, la quatrième extrémité distale du tube interne (500) dépasse de la première extrémité distale du tube externe (200) et est reliée à l'élément de guidage (600) ;
comprenant en outre au moins un anneau de développement (520) disposé à la quatrième extrémité distale du tube interne (500).

10. Système de pose d'implant (10) selon la revendication 1, comprenant en outre un élément de guidage (600), et le tube interne (500) a une quatrième extrémité proximale et une quatrième extrémité distale opposées l'une à l'autre, la quatrième extrémité distale du tube interne (500) dépasse de la première extrémité distale du tube externe (200) et est reliée à l'élément de guidage (600) ;
dans lequel la première extrémité proximale du tube externe (200) est reliée à une paroi externe du tube de poussée (300) par l'intermédiaire d'un ensemble de connexion de tube externe (210), et le tube externe (200) peut coulisser le long de la paroi externe du tube de poussée (300) ;
dans lequel l'ensemble de connexion de tube externe (210) comprend un connecteur de tube externe (211), une première bague d'étanchéité (212) et un premier bouchon d'étanchéité (213) ; le connecteur de tube externe (211) a un premier trou de connexion dont une extrémité est reliée de manière fixe au tube externe (200), une autre extrémité est manchonnée sur le tube de poussée (300) ; et la première bague d'étanchéité (212) est manchonnée sur le tube de poussée (300) et est en connexion étanche avec le tube externe (200) lorsque le premier bouchon d'étanchéité (213) est serré.

11. Système de pose d'implant (10) selon la revendication 10, dans lequel le connecteur de tube externe (211) est en outre défini avec une première rainure pour segment d'arrêt, le premier élément coulissant interne (120) est pourvu d'un premier segment d'arrêt (123), et la première rainure pour segment d'arrêt est reliée de manière amovible au premier segment d'arrêt (123).

12. Système de pose d'implant (10) selon la revendication 10, dans lequel la seconde extrémité proximale du tube de poussée (300) est pourvue d'un ensemble de connexion de tube de poussée (320) comprenant un connecteur de tube de poussée (321), dans lequel le connecteur de tube de poussée (321) est disposé à la seconde extrémité proximale du tube de poussée (300), et le connecteur de tube de poussée (321) est défini avec un second trou de connexion à travers lequel le connecteur de tube de poussée (321) est manchonné sur le tube interne (500) et peut être coulissé le long du tube interne (500) ;
dans lequel l'ensemble de connexion de tube de poussée comprend en outre une seconde bague d'étanchéité (322) et un second bouchon d'étanchéité (323), la seconde bague d'étanchéité (322) est manchonnée sur le tube interne (500), le second bouchon d'étanchéité (323) est utilisé pour fixer la seconde bague d'étanchéité (322) de sorte que le connecteur de tube de poussée (321) est en connexion étanche avec le tube interne (500).

13. Système de pose d'implant (10) selon la revendication 10, dans lequel la seconde extrémité proximale du tube de poussée (300) est pourvue d'un ensemble de connexion de tube de poussée (320) comprenant un connecteur de tube de poussée (321), dans lequel le connecteur de tube de poussée (321) est disposé à la seconde extrémité proximale du tube de poussée (300), et le connecteur de tube de poussée (321) est défini avec un second trou de connexion à travers lequel le connecteur de tube de poussée (321) est manchonné sur le tube interne (500) et peut être coulissé le long du tube interne (500) ;
dans lequel le connecteur de tube de poussée (321) est défini avec une seconde rainure pour segment d'arrêt, le second élément coulissant interne (130) est pourvu d'un second segment d'arrêt (133), et la seconde rainure pour segment d'arrêt est reliée de manière amovible au second segment d'arrêt (133).

14. Système de pose d'implant (10) selon la revendication 12, comprenant un premier tube de perfusion (710) et un second tube de perfusion (810) ; la quatrième extrémité proximale du tube interne (500) fait saillie à partir de la seconde extrémité proximale du tube de poussée (300) et est en communication avec le premier tube de perfusion (710) ; le connecteur de tube externe (211) est pourvu d'un premier connecteur de dérivation, et un troisième trou de connexion est défini dans le premier connecteur de dérivation et est en communication avec le second tube de perfusion (810) ; ou,
le connecteur de tube de poussée (321) est pourvu d'un second connecteur de dérivation (324), et un quatrième trou de connexion est défini dans le second connecteur de dérivation (324) et est en communication avec le second tube de perfusion (810) ;
dans lequel la quatrième extrémité proximale du tube interne (500) est pourvue d'un ensemble de connexion de tube interne (510) comprenant un premier manchon de connexion (511), un second manchon de connexion (512) et un bouchon de verrouillage (513) ; le premier manchon de connexion (511) est utilisé pour connecter le tube interne (500) et le premier tube de perfusion (710), le second manchon de connexion (512) est manchonné sur le premier manchon de connexion (511) et est utilisé pour fixer la quatrième extrémité proximale du tube interne (500), et le bouchon de verrouillage (513) est utilisé pour verrouiller le second manchon de connexion (512), le premier manchon de connexion (511) et le premier tube de perfusion (710) en position.

15. Système de pose d'implant (10) selon l'une quelconque des revendications 1 à 3, dans lequel le premier élément coulissant interne (120) et le second élément coulissant interne (130) ont la forme de deux structures creuses en forme de tube disposées coaxialement l'une par rapport à l'autre.
